# EUROPÄISCHE PATENTSCHRIFT

(11) **EP 0 909 765 B1**
(45) Veröffentlichungstag und Bekanntmachung des Hinweises auf die Patenterteilung: **29.10.2008**
(21) Anmeldenummer: 98115797.7
(22) Anmeldetag: 21.08.1998
(51) Int. Cl.: C07K 14/585, A61K 38/23

(54) **Calicitonin-Analoga mit stark erhöhter hypocalcämischer Wirkung in vivo**
Calcitonin analogues having increased hypocalcaemic activity in vivo
Analogues de la calcitonine ayant une activité hypocalcémique acrrue in vivo

(30) Priorität: 21.08.1997 DE 19736457
(43) Veröffentlichungstag der Anmeldung: 21.04.1999
(73) Patentinhaber: Fraunhofer-Gesellschaft zur Förderung der angewandten Forschung e.V., 80686 München (DE)
(72) Erfinder: Kapurniotu, Afroditi, Dr., 72074 Tübingen (DE); Bernhagen, Jürgen, Dr., 72074 Tübingen (DE); Brunner, Herwig, Prof. Dr., 70569 Stuttgart (DE)

(56) Entgegenhaltungen:
- DE-A- 4 431 121
- YANG WEI ET AL: "Systematic analysis of the Saccharomyces cerevisiae alpha-factor containing lactam constraints of different ring size." BIOCHEMISTRY, Bd. 34, Nr. 4, 1995, Seiten 1308-1315, XP001015419 ISSN: 0006-2960
- SZEWCZUK ZBIGNIEW ET AL: "Conformationally restricted thrombin inhibitors resistant to proteolytic digestion." BIOCHEMISTRY, Bd. 31, Nr. 38, 1992, Seiten 9132-9140, XP001015422 ISSN: 0006-2960
- KAPURNIOTU APHRODITE ET AL: "Rational design, conformational studies and bioactivity of highly potent conformationally constrained calcitonin analogues." EUROPEAN JOURNAL OF BIOCHEMISTRY, Bd. 265, Nr. 2, Oktober 1999 (1999-10), Seiten 606-618, XP002174066 ISSN: 0014-2956

## Beschreibung

Die vorliegende Erfindung bezieht sich auf Calcitonine und Calcitonin-Derivate mit hypocalcämischem Effekt. Derartige Calcitonine und Calcitonin-Derivate werden insbesondere im Bereich der pharmazeutischen Industrie und im Bereich der Medizin, beispielsweise zur Behandlung von Osteoporose, der Paget'schen Krankheit oder der Hypercalcämie eingesetzt.

Calcitonine sind Peptidhormone, die aus 32 Aminosäuren bestehen. Aufgrund ihres hypocalcämischen Effektes und der durch sie bewirkten Inhibition des Knochenabbaus besitzen sie große pharmakologische Bedeutung. Sie werden therapeutisch eingesetzt zur Behandlung von Osteoporose, der Paget'schen Krankheit oder der Hypercalciämie. Verwendet werden dabei insbesondere die Calcitonine des Menschen (hCt), des Schweines (pCt) oder von ultimobranchialen Spezies, wie dem Lachs (sCt) oder dem Aal (eCt).

Die Calcitonine oder deren Derivate von ultimobranchialen Spezies besitzen in vivo eine 20- bis 50fach höhere Wirksamkeit als das humane Calcitonin. Daher werden diese Calcitonine vorzugsweise zu therapeutischen Zwecken eingesetzt. Die Calcitonine der ultimobranchialen Spezies unterscheiden sich jedoch in ihrer Aminosäuren-Abfolge erheblich von dem Peptid des humanen Calcitonins. Beispielsweise unterscheidet sich das Lachs-Calcitonin in 16 der 32 Aminosäuren von dem humanen Calcitonin. Dennoch wird es bis heute angewandt, da aufgrund seiner erheblich höheren Wirkung die Dosierung zu therapeutischen Zwecken niedriger gehalten werden kann.

Nachteilig an Calcitoninen ultimobranchialer Spezies ist jedoch, daß sie aufgrund der Aminosäuren-Unterschiede eine Immunreaktion im Menschen hervorrufen. Diese hohe Antigenizität führt zur Antikörperbildung gegen das jeweilige therapeutisch eingesetzte Calcitonin, oftmals bereits sechs Monate nach Applikationsbeginn. Diese hohe Antigenizität führt anschließend zu sekundärer Resistenz und Desensitisierung gegenüber dem eingesetzten Calcitonin. Dies erfordert andererseits die Applikation höherer Dosen, welche wiederum zu höherer Antikörperbildung und damit sekundärer Resistenz sowie zu weiteren Nebeneffekten (Gegenanzeigen) führen.

Von den bekannten Calcitoninen sind weitere, gegenüber der nativen Form aktivere Derivate bekannt. Beispielsweise führt ein Austausch von drei bis fünf Aminosäure-Resten auf der hydrophoben Seite der potentiellen α-helikalen Region (8-22) von hCt durch Leucine zu aktiveren Calcitonin-Analoga. Die Aktivität der Calcitonin-Derivate wird dabei auf eine enge Beziehung zwischen einer potentiellen amphiphilen αhelikalen Region zwischen den Aminosäureresten 8 und 22 und der biologischen Wirkung des Moleküls, andererseits auf die Konformationsflexibilität und die "durch-den-Raum"-Wirkungen verschiedener Molekül-Regionen zurückgeführt.

Abgesehen von sCt und α-Aminosuberinsäure-1,7-eCt finden diese Analoga jedoch bisher keine weitere klinische Anwendung zur Behandlung der oben erwähnten Krankheiten.

Da aufgrund der hohen proteolytischen Abbaurate (in-vivo-Halbwertszeit) der zur Zeit therapeutisch eingesetzten-Calcitonine verhältnismäßig hohe Mengen verabreicht werden müssen, wobei dies - wie oben beschrieben - wiederum zu einer schnellen Antikörperbildung (sekundäre Resistenz) und eventuellen Nebeneffekten (Gegenanzeigen) führt, wird der Erhöhung der Bioaktivität oder Proteolyse-Resistenz große Bedeutung beigemessen.

Derartige konformationsstabilisierte Analoga von hCt mit erhöhter hypocalcämischer Wirkung sind aus der DE 44 31 121 A1 bekannt. In dieser Schrift sind hCt-Derivate beschrieben, bei denen durch die Einführung einer kovalenten Lactamverbrückung zwischen den Aminosäuren in Positionen 17 und 21 eine 20-gliedrige Ringstruktur erzeugt wird, beispielsweise indem die Aminosäure in Position 17 Asparaginsäure und die Aminosäure in Position 21 Lysin ist. Diese hCt-Analoga besitzen eine erhöhte Konformationsstabilität und eine erhöhte hypocalcämische Wirkung.

Diese hypocalcämische Wirkung ist jedoch noch nicht ausreichend, um eine therapeutische Anwendung dieser hCt-Analoga zur Behandlung der oben beschriebenen Krankheiten zu ermöglichen.

Aufgabe der vorliegenden Erfindung ist es, Calcitonine oder Calcitonin-Derivate zur Verfügung zu stellen, die eine erhöhte Konformationsstabilität aufweisen und eine hohe biologische Aktivität besitzen.

Diese Aufgabe wird durch die Calcitonine und Calcitonin-Derivate nach dem Oberbegriff des Anspruches 1 in Verbindung mit seinen kennzeichnenden Merkmalen gelöst.

Die erfindungsgemäßen Calcitonine und Calcitonin-Derivate besitzen eine Verbrückung zwischen den Aminosäuren an den den Positionen 17 und 21 des humanen Calcitonins entsprechenden Positionen. An diesen Positionen sind derartige Aminosäuren eingebaut, daß durch die Verbrückung ein Ring gebildet wird, der 18 oder 19 Glieder aufweist. Im Gegensatz zu den aus dem Stand der Technik bekannten 20-gliedrigen Ringstruktur sind die erfindungsgemäßen Calcitonine bezüglich ihrer Konformation stabilisierter, so daß der proteolytische Abbau verlangsamt ist. Weiterhin weisen diese erfindungsgemäßen Calcitonine und Calcitonin-Derivate eine sehr hohe Aktivität auf, wodurch eine geringe Dosierung möglich ist. Dadurch, daß die Sequenz sehr ähnlich zum humanen Molekül ist, tritt keine Immunreaktion (keine sekundäre Resistenz) auf. Insgesamt wurde festgestellt, daß die Ringverkleinerung im Vergleich zum Stand der Technik zu einer sehr hohen Aktivität führt, so daß sich eine 19-gliedrige oder 18-gliedrige Ringstruktur als Leitstruktur für weitere potente Calcitonin-Analoga über die in den Beispielen beschriebenen hinaus eignet.

Vorteilhafte Weiterbildungen der erfindungsgemäßen Calcitonine und Calcitonin-Derivate werden in den abhängigen Ansprüchen beschrieben.

Die Aminosäuren in den Positionen 17 und 21 können durch geeignete Linker oder bei entsprechender Auswahl der Aminosäure über eine Lactambrücke verbrückt sein. Besonders vorteilhafte Eigenschaften besitzen Calcitonine, bei denen sich an den genannten Positionen Asparaginsäure und Ornithin statt Asparagin und Threonin im humanen Calcitonin befinden. Dieses Calcitonin ist 360-mal aktiver als das ursprüngliche humane Calcitonin und immer noch 3-mal aktiver als Calcitonin aus Lachs (sCt).

Auch durch die Einfügung von Glutaminsäure und α,γ-Diaminobuttersäure statt Asparagin und Threonin an den Positionen 17 und 21 wird ein 19-gliedriger Ring und ein hochaktives Analog erzeugt.

Ein erfindungsgemäßes Calcitonin-Derivat mit 18-gliedrigem Ring ergibt sich durch den Ersatz der Aminosäuren Asparagin und Threonin an den Positionen 17 bzw. 21 durch Asparaginsäure bzw. α,γ-Diaminobuttersäure. Dieses Calcitonin-Derivat ist ca. 30-mal aktiver als das ursprüngliche humane Calcitonin.

In dieser Erfindung werden weitere hochaktive Analoga mit einem 19- oder 18-gliedrigen Ring gegeben einschließlich der folgenden Aminosäurepaare in den Positionen 17 und 21 des Calcitonins 21: Asparaginsäure und α,γ-Diaminobuttersäure oder α,γ-Diaminobuttersäure und Asparaginsäure oder Glutaminsäure und α,γ- Diaminobuttersäure oder α,γ-Diaminobuttersäure und Glutaminsäure oder 2-Aminoadipinsäure und Serin oder Serin und 2-Aminoadipinsäure oder Glutaminsäure und Serin oder Serin und Glutaminsäure.

Die genannte Erhöhung der Stabilität und Aktivität durch die Einführung des erfindungsgemäßen 18-gliedrigen bzw. 19-gliedrigen Ringsystems ergibt sich jedoch nicht nur bei humanem Calcitonin, sondern auch bei den Calcitoninen des Schweines oder der ultimobranchialen Spezies wie Lachs oder Aal oder deren bisher bekannten Analoga. Beispielsweise kann die Aktivität eines bekannten hochaktiven Analogons, bei dem die Positionen 1 und 7 durch einen α-Aminosuberinsäure-Rest ersetzt sind, durch die Einführung eines 18-gliedrigen oder 19-gliedrigen Ringes wie oben beschrieben weiter gesteigert werden. Auch bei bereits bekannten Analoga ergibt sich also durch die Einführung der erfindungsgemäßen 18- bzw. 19-gliedrigen Ringstruktur eine Verbesserung der Stabilität und Aktivität.

Im folgenden werden einige Beispiele der erfindungsgemäßen Calcitonine und Calcitonin-Derivate sowie ihre Herstellung gegeben. Es zeigen:
- Fig. 1: die Primärstruktur des humanen Calcitonins nach dem Ein-Buchstaben-Code für Aminosäu- ren;
- Fig. 2: die Primärstruktur des Cyclo^{17,21}- [Asp¹⁷,Orn²¹]-hCt nach dem Ein-Buchstaben- Code für Aminosäuren.

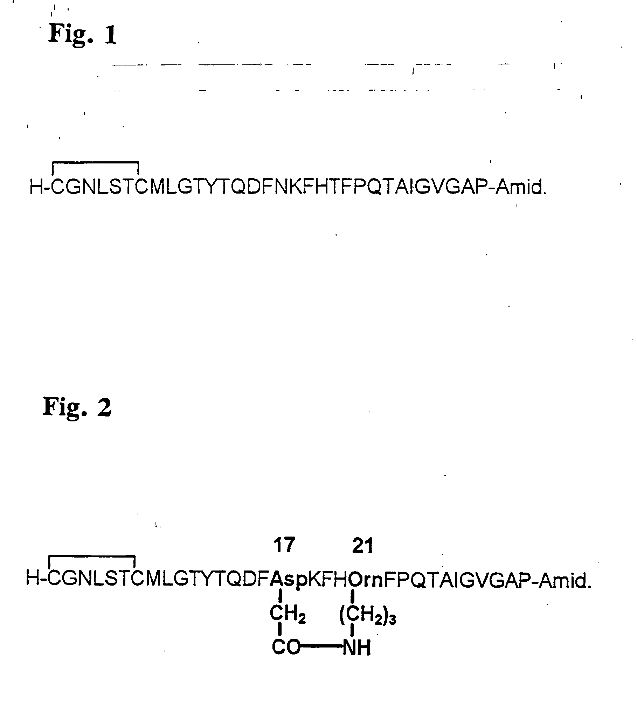

Fig. 1 zeigt die Primärstruktur des humanen Calcitonins. An den Positionen 17 und 21 befindet sich Asparagin bzw. Threonin.

Fig. 2 zeigt ein erfindungsgemäßes humanes Calcitonin, bei dem die Aminosäuren in Positionen 17 und 21 durch Asparaginsäure bzw. Ornithin ersetzt und ihre Seitenketten zur Erzeugung eines 19-gliedrigen Ringes über eine Lactam-Verbrückung kovalent verbunden sind. Dabei ist die Aminosäuresequenz im Ein-Buchstaben-Code geschrieben, jedoch die Aminosäuren 17 und 21 zur besseren Veranschaulichung nach dem Drei-Buchstaben-Code und ihre über eine Lactam-Brücke verbundenen Seitenketten mit ihren Strukturformeln dargestellt. Weiterhin ist die S-S-Verbrückung zwischen Cys¹ und Cys⁷ angedeutet.

Dieses erfindungsgemäße Calcitonin besitzt eine Aktivität, die im Maus-in-vivo-Test um das 363-fache über der Aktivität des in-Fig. 1 dargestellten humanen Calcitonins liegt. Vorteilhaft an diesem in Fig. 2 dargestellten Calcitonin sind folglich seine hohe Aktivität und seine hohe konformationelle Stabilität, weshalb eine geringe Dosierung zu therapeutischen Zwecken möglich ist. Dies führt dazu, daß keine sekundären Resistenzen zu erwarten sind. Weiterhin handelt es sich hierbei um das erste humane Calcitonin, das eine ausreichend hohe Aktivität für die therapeutische Anwendung aufweist. Da die Primärstruktur neben dem Austausch von Asparagin gegen Asparaginsäure nur in einer weiteren Aminosäure (Ornithin statt Threonin) geringfügig von der Primärstruktur des nativen humanen Calcitonins abweicht, sind nur sehr geringe bis keine Immunreaktionen zu erwarten, und daher können Nebenwirkungen (wie z.B. sekundäre Resistenz) weitgehend vermieden werden.

Statt Asparaginsäure und Ornithin in den Positionen 17 bzw. 21 wird auch durch die Verwendung von Glutaminsäure bzw. α,γ-Diaminobuttersäure in diesen Positionen ein 19-gliedriger Ring erreicht, wodurch sich ein Derivat des humanen Calcitonins ergibt, das ebenfalls eine hohe Aktivität und hohe Stabilität bei gleichzeitig äußerst geringer Immunreaktion aufweist.

Als Beispiel für einen 18-gliedrigen Ring wird hier das Calcitonin-Derivat gegeben, bei dem in den Positionen 17 bzw. 21 Asparaginsäure bzw. α,γ-Diaminobuttersäure angeordnet sind. Hierdurch ergibt sich ein Derivat des humanen Calcitonins mit einem 18-gliedrigen Ring, das ebenfalls eine hohe Aktivität, hohe konformationelle Stabilität aufweist, wobei aufgrund der geringen Unterschiede zur Primärsequenz des nativen humanen Calcitonins nur eine geringe Immunreaktion auftritt.

Die genannten Verbesserungen der Aktivität und Stabilität können ebenfalls bei bereits bekannten Calcitoninen, beispielsweise des Lachses, des Aales oder des Schweines, oder deren bereits bekannten Analoga erzielt werden.

Die erfindungsgemäßen Calcitonine und Calcitonin-Derivate werden im wesentlich peptidsynthetisch hergestellt.

Im folgenden wird beispielhaft die Synthese und Aufreinigung, Charakterisierung und Testung der biologischen Aktivität des Cyclo^{17,21}-[Asp¹⁷,Orn²¹]-hCt aus Fig. 1 beschrieben.

### Synthese und Aufreinigung

Für die Synthesen der in dieser Erfindung beschriebenen konformationsstabilisierten Calcitonin-Analoga wird die Festphasenmethode der Peptidsynthese in Verbindung mit einer Methode zur Herstellung von cyclischen, durch die Seitenketten lactamverbrückten Peptide, direkt auf den polymeren Träger (Harz) nach Felix A.M. et al. (Int. J. Pept. Prot. Res. 32 (1988), 441-445) eingesetzt. Die Synthese des hCt-Analogons Cyclo^{17,21}- [Asp¹⁷,Orn²¹]-hCt wird im folgenden detailliert beschrieben:

Zunächst wird Peptidharz (1) (N-Boc-(hCt(22-32))-MBHA) nach gängigen Methoden der Festphasenpeptid-Synthese wie folgt hergestellt:

4 g eines p-Methylbenzhydrylamin-Harzes, welches in der Form eines HCl-Salzes vorliegt, mit einer Beladung von 0,65 mmol/g wird in einer Schüttelente zunächst mit Triethylamin in DMF (Dimethylformamid) neutralisiert und mit DMF und Dichlormethan (DCM) gewaschen. Es folgt die Zugabe von Boc-Pro-OH und DCC in dreifachem Überschuß (7,2 mmol) in Dichlormethan (30 ml), und die Reaktionssuspension wird 18 h geschüttelt. Danach wird das Polymer mit DCM, iProOH, DCM, DMF gewaschen und die Beladung des Harzes nach der Pikrinsäure-Methode bestimmt (0,62 mmol/g). Die restlichen freien Aminogruppen werden mittels Essigsäureanhydrid (26 mmol) und Pyridin (26 mmol) in DCM (30 ml) (1 h) acetyliert und wie üblich gewaschen. Für die anschließende Verlängerung der Peptidkette wird die TBTU-Methode (3-facher Überschuß an geschützter Aminosäure) in DMF verwendet. Die Vollständigkeit der Kupplungen wird mittels des Kaiser-Testes überprüft. Für Ile²⁷ und Phe²² werden doppelte Kupplungen durchgeführt. Die Aminosäure-Derivate sind N-Boc-geschützt, und Thr wird als Boc-Thr(Bz) eingesetzt. Für die Abspaltung der Boc-Gruppe wird 50 % TFA (Trifluoressigsäure) in DCM verwendet (30 min). Das wie oben beschrieben erhaltene Peptidharz 1 hat eine Substitution von 0,57 mmol/g und wird durch Aminosäureanalyse und FAB-MS nach Abspaltung des Peptids aus einem kleinen Teil des Peptidharzes charakterisiert. Ein Teil des wie oben beschrieben erhaltenen Peptidharzes 1 (500 mg; 0,17 mmol) wird mit Boc-Orn(Fmoc) gekuppelt (1 mmol) und anschließend mit Essigsäureanhydrid (1 mmol) in Gegenwart von Pyridin (1 mmol)acetyliert. Das erhaltene "verdünnte" (Beladung 0,21 mmol/g Harz) Peptidharz wird dann bis zum Aminosäure-Rest Asp¹⁷ verlängert (Kupplungsmethode wie beschrieben für 1). Dabei werden N-Boc-geschützte Aminosäure-Derivate verwendet, und His²⁰ und Lys¹⁸ werden als Boc-Lys(2ClZ) und Boc-His(Bom) eingesetzt. Die N^{α}-Fmoc-Gruppe von Orn²¹ und der Fluorenylmethylester(OFm)-Schutz des β-Carboxyls von Asp¹⁷ werden durch Behandlung mit einer 20%igen Piperidinlösung in DMF (1 x 1 min; 1 x 28 min) nach der Methode von Felix et al. (Felix, A.M. et al., Int. J. Pept. Protein Res. 32 (1988) 441-454) abgespalten. Die Lactam-Verbrückung zwischen den von Schutzgrupopen befreiten Seitenketten von Asp¹⁷ und Orn²¹ erfolgt dann durch die Zugabe von BOP (Benzotriazol-1-yl-oxy-tris-(dimethylamino)-phosphoniumhexafluorophosphat) (0,3 mmol) und DIEA (0,34 mmol) in DMF (50 ml) zum Peptidharz und anschließendes Schütteln (für 4 h). Die Cyclisierung wird durch den Kaiser-Test verfolgt.

Nach dem Entfernen der Kupplungsreagenzien wird das Peptidharz wie oben beschrieben acetyliert. Die weitere Verlängerung des so erhaltenen Peptidharzes mit N-Boc-geschützten Aminosäuren erfolgt nach der TBTU-Kupplungsmethode. Die Seitenketten der trifunktionellen Aminosäuren werden wie folgt geschützt:

Asp(β-OcHx), Cys(S-p-Mb), Glu(γ-OBzl), Thr(Bzl) und Tyr(2BrZ). 100 mg des so erhaltenen NH₂-Cyclo^{17,21}-[Asp¹⁷,Orn²¹]-hCt-MBHA werden mit einer Mischung aus HF/Anisol/Dimethylsulfid/p-Thiokresol im Verhältnis 10/1/1/0,2 (v/v/v/w), welche auch Cystein in einer Endkonzentation von 0,27 M enthält, behandelt (30 min bei -20 °C und 1 h auf 0 °C). Das von Schutzgruppen befreite und vom Harz abgespaltene Rohprodukt wird durch Zugabe von Ether ausgefällt, mit Ether dreimal gewaschen und in 10 % Essigsäure extrahiert (4 x 20 ml) und lyophilisiert. Es folgt Luftoxidation dieses Rohproduktes zur Schließung der Disulfidbrücke in einer 0,1 M NH₄HCO₃-Lösung bei hoher Verdünnung (10⁻⁴ M) im Dunkeln (24 h). Danach wird die Lösung lyophilisiert. Das so erhaltene Rohprodukt wird durch präparative Umkehrphasen-HPLC auf einer C¹⁸-Säule gereinigt. Es wird ein Gradient von 30-70 % B über 30 min verwendet, wobei A aus 0,058 % TFA in Wasser und B aus 0,05 % TFA in 90 % Acetonitril besteht. Die Detektion der Peptide erfogt bei 220 nm.

Die Synthese und Aufreinigung der anderen hCt-Analoga mit 19- bzw. 18-gliedriger Ringstruktur erfolgt analog.

### Charakterisierung

Die Reinheit des so erhaltenen Cyclo^{17,21}-[Asp¹⁷,Orn²¹]-hCt wurde durch Aminosäure-Analyse bestimmt. Zum Identitätsnachweis wurde zudem MALDI-TOF-Massenspektrometrie oder Elektrospray-Massenspektrometrie eingesetzt. Die Bestimmung der Konformation der Analoga erfolgte durch Circulardichroismus-Spektropolarimetrie.

### Testung der biologischen Aktivität

Die biologische Aktivität der Analoga wurde mittels eines hypocalcämischen Testes in vivo in BALB/c-Mäusen bestimmt.

Tabelle 1 zeigt die Dosis-Wirkungsbestimmung des hypocalcämischen Effektes von Cyclo^{17,21}-[Asp¹⁷ ,Orn²¹]-hCt im Vergleich zu nativem humanen Calcitonin (hCt) und Calcitonin aus Lachs (sCt). Die biologische Aktivität ist als Prozent [%] Aktivität des maximal in diesem Test erreichbaren hypocalcämischen Effektes, wie er durch 2 µg hCt erreicht wird, dargestellt.

**Tabelle 1**

| Konzentration der Peptide | Cyclo^{17,21}-[Asp¹⁷, Orn²¹]-hCt | hCt | sCt |
|---|---|---|---|
| (ng/ml) | (%) | (%) | (%)] |
| | | | |
| 10 | 86,8 | 35,2 | - |
| 1 | 76,3 | 15,8 | 63,2 |
| 0,1 | 68,4 | 5,8 | 51,6 |
| 0,01 | 36,8 | - | 21,1 |

## Patentansprüche

1. Calcitonine und Calcitonin-Derivate mit einer Verbrückung zwischen den Aminosäuren an den den Positionen 17 und 21 des humanen Calcitonins entsprechenden Positionen,
**dadurch gekennzeichnet ,**
**daß** in diesen Positionen solche Aminosäuren angeordnet sind, daß der durch die Verbrückung gebildete Ring 18 oder 19 Glieder aufweist.

2. Calcitonine und Calcitonin-Derivate nach Anspruch 1,
**dadurch gekennzeichnet, daß** die Aminosäuren in den Positionen 17 und 21 durch eine Lactambrücke verbrückt sind.

3. Calcitonine und Calcitonin-Derivate nach Anspruch 1,
**dadurch gekennzeichnet, daß** die Aminosäuren in den Positionen 17 und 21 durch geeignete Linker verbrückt sind.

4. Calcitonine und Calcitonin-Derivate nach mindestens einem der vorhergehenden Ansprüche, **dadurch gekennzeichnet, daß** sich an den Positionen 17 bzw. 21 Asparaginsäure bzw. Ornithin oder Ornithin bzw. Asparaginsäure befinden.

5. Calcitonine und Calcitonin-Derivate nach mindestens einem der Ansprüche 1 bis 3,
**dadurch gekennzeichnet, daß** sich an den Positionen 17 und 21 Paare von Asparaginsäure und α,γ-Diaminobuttersäure oder α,γ-Diaminobuttersäure und Asparaginsäure oder Glutaminsäure und α,γ-Diaminobuttersäure oder
α,γ-Diaminobuttersäure und Glutaminsäure oder 2-Aminoadipinsäure und Serin oder
Serin und 2-Aminoadipinsäure oder
Glutaminsäure und Serin oder
Serin und Glutaminsäure befinden.

6. Calcitonine und Calcitonin-Derivate nach mindestens einem der vorhergehenden Ansprüche, **dadurch gekennzeichnet, daß** ihre Aminosäuresequenz im übrigen der Sequenz bekannter Calcitonine bzw. bekannter Calcitoninanaloga, insbesondere des Menschen, des Lachses, des Aals oder des Schweines entspricht.

7. Calcitonine und Calcitonin-Derivate nach mindestens einem der vorhergehenden Ansprüche, **dadurch gekennzeichnet, daß** die Cysteinreste in den Positionen 1 und 7 durch einen α-Aminosuberinsäurerest ersetzt sind.

## Claims

1. Calcitonins and calcitonin derivatives with a bridge between the amino acids at the positions corresponding to the positions 17 and 21 of human calcitonin, **characterized in that** such amino acids are arranged in these positions that the ring formed by the bridge has 18 or 19 members.

2. Calcitonins and calcitonin derivatives according to Claim 1, **characterized in that** the amino acids in the positions 17 and 21 are bridged by a lactam bridge.

3. Calcitonins and calcitonin derivatives according to Claim 1, **characterized in that** the amino acids in the positions 17 and 21 are bridged by suitable linkers.

4. Calcitonins and calcitonin derivatives according to at least one of the preceding Claims, **characterized in that** aspartic acid and ornithine or ornithine and aspartic acid respectively are present at the positions 17 and 21.

5. Calcitonins and calcitonin derivatives according to at least one of Claims 1 to 3, **characterized in that** pairs of aspartic acid and α,γ-diaminobutyric acid or α,γ-diaminobutyric acid and aspartic acid or glutamic acid and α,γ-diaminobutyric acid or α,γ-diaminobutyric acid and glutamic acid or 2-aminoadipic acid and serine or serine and 2-aminoadipic acid or glutamic acid and serine or serine and glutamic acid are present at the positions 17 and 21.

6. Calcitonins and calcitonin derivatives according to at least one of the preceding Claims, **characterized in that** their amino acid sequence otherwise corresponds to the sequence of known calcitonins or known calcitonin analogues, in particular of humans, of salmon, of eels or of pigs.

7. Calcitonins and calcitonin derivatives according to at least one of the preceding Claims, **characterized in that** the cysteine residues in the positions 1 and 7 are replaced by an α-aminosuberic acid residue.

## Revendications

1. Calcitonines et dérivés de calcitonine présentant un pont établi entre les acides aminés situés dans des positions correspondant aux positions 17 et 21 de la calcitonine humaine,
**caractérisés en ce que** ces acides aminés sont agencés dans ces positions de sorte que le cycle formé par pontage présente 18 ou 19 éléments.

2. Calcitonines et dérivés de calcitonine selon la revendication 1, **caractérisés en ce que** les acides aminés situés dans les positions 17 et 21 sont reliés par un pont de type lactame.

3. Calcitonines et dérivés de calcitonine selon la revendication 1, **caractérisés en ce que** les acides aminés situés dans les positions 17 et 21 sont pontés par le biais d'un lieur approprié.

4. Calcitonines et dérivés de calcitonine selon au moins l'une quelconque des revendications précédentes,
**caractérisés en ce que** l'on trouve l'acide aspartique et l'ornithine ou l'ornithine et l'acide aspartique dans les positions respectivement 17 et 21.

5. Calcitonines et dérivés de calcitonine selon au moins l'une quelconque des revendications 1 à 3,
**caractérisés en ce que** l'on trouve dans les positions 17 et 21 des paires d'acide aspartique et d'acide α,γ-diaminobutyrique, ou d'acide α,γ-diaminobutyrique et d'acide aspartique, ou d'acide glutamique et d'acide α,γ-diaminobutyrique, ou d'acide α,γ-diaminobutyrique et d'acide glutamique, ou d'acide 2-aminoadipique et de sérine, ou de sérine et d'acide 2-aminoadipique, ou d'acide glutamique et de sérine ou de sérine et d'acide glutamique.

6. Calcitonines et dérivés de calcitonine selon au moins l'une quelconque des revendications précédentes,
**caractérisés en ce que** leur séquence d'acides aminés correspond, dans l'ensemble, à la séquence de calcitonines connues ou respectivement d'analogues de calcitonine connus, en particulier, de l'être humain, du saumon, de l'anguille ou du porc.

7. Calcitonines et dérivés de calcitonine selon au moins l'une quelconque des revendications précédentes,
**caractérisés en ce que** les radicaux de cystéine situés dans les positions 1 et 7 sont remplacés par un radical d'acide α-aminosubérique.
